(19) Europäisches Patentamt
European Patent Office
Office européen des brevets

(11) Numéro de publication: **0 179 324 B1**

(12) **FASCICULE DE BREVET EUROPEEN**

(45) Date de publication de fascicule du brevet: **16.10.91**

(51) Int. Cl.[5]: **C07H 13/04**, A61K 31/70, A61L 2/16, C12Q 1/04, //G01N33/555,A23C9/152

(21) Numéro de dépôt: **85112511.2**

(22) Date de dépôt: **03.10.85**

(54) **Composition anti-bactérienne.**

(30) Priorité: **26.10.84 CH 5136/84**

(43) Date de publication de la demande: **30.04.86 Bulletin 86/18**

(45) Mention de la délivrance du brevet: **16.10.91 Bulletin 91/42**

(84) Etats contractants désignés: **AT BE DE FR GB IT LU NL SE**

(56) Documents cités:
**EP-A- 0 089 940**

**FEMS MICROBIOLOGY LETTERS, vol. 20, 1983, pp 237-242; Elsevier A. PIERCE-CRETEL: "Oligosaccharide structural specificity of the soluble agglutinin released from guinea pig colonic epithelial cells"**

**CARBOHYDRATE RESEARCH, vol. 120, 1983, pp 283-292; Elsevier Science Publihers B.V., Amsterdam, NL Y.KATAGIRI et al.: Structural requirements for the binding of high-man-nose-type glycopeptides to immobilized pokeweed pa-2 lectin".**

(73) Titulaire: **SOCIETE DES PRODUITS NESTLE S.A.**
**Case postale 353**
**CH-1800 Vevey(CH)**

(72) Inventeur: **Neeser, Jean-Richard**
**Rue du Temple 34**
**CH-1010 Lausanne(CH)**
Inventeur: **Würsch, Pierre**
**Ch. de la Crausaz 64**
**CH-1814 La Tour-de-Peilz(CH)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

L'invention concerne une composition anti-bactérienne relative à des bactéries pathogènes pourvues de fimbriae de type I, contenant comme constituants actifs des glycopeptides et/ou des oligosaccharides ainsi qu'un procédé de préparation desdits constituants actifs.

Il y a peu de doute que la plupart des infections naturelles débutent par l'adhérence de l'agent pathogène aux cellules épithéliales des muqueuses, qui permet ensuite son implantation et la colonisation du tissu animal. Dans le processus d'infection par les bactéries pourvues de structures protéiniques appelées "fimbriae de type I", bactéries dont l'adhérence aux cellules animales est dite "sensible au mannose", ces structures reconnaissent des récepteurs spécifiques qui sont des groupements glucidiques complexes, vraisemblablement des chaînes d'oligomannosides faisant partie des glycoconjugués présentés par la surface des membranes cellulaires.

On a montré récemment (réf. 1 ci-après) que le phénomène d'adhérence des bactéries pourvues de fimbriae de type I sur les cellules éphithéliales ainsi que leur capacité à provoquer l'hémagglutination d'érythrocytes de cobaye, peuvent être inhibés en présence de certains oligosaccharides ressemblant à la structure spécifique des récepteurs membranaires, la bactérie étant ainsi leurrée en fixant préférentiellement les oligosaccharides en question. Ces oligosaccharides ont été, soit obtenus par voie de synthèse, soit isolés à partir d'urines de patients présentant des déficiences enzymatiques.

Dans le même ordre d'idées, la demande de brevet européen publiée No. 89940 par exemple, concerne une composition contenant la structure Galα1→3Gal obtenue par voie chimique capable d'inhiber "in vitro" l'adhérence de Escherichia Coli K88+ sur les cellules intestinales des porcelets.

L'invention a pour but de fournir une composition contenant comme constituant actif un glycopeptide et/ou un oligosaccharide d'origine végétale ou un glycopeptide d'ovalbumine otenu par voie enzymatique ayant la capacité de remplacer le récepteur normal que reconnaissent les fimbriae de type I et ainsi d'inhiber ou de renverser l'adhérence des bactéries pathogènes pourvues de ces fimbriae sur les cellules animales.

Dans FEMS. Microb. Lett. 20,237-42(1983), A. Pierce-Cretel et coll. divulguent que les cellules épithéliales du colon de cochon d'Inde libèrent une lectine capable d'agglutiner la bactérie S. flexeneri 1b. La lectine libérée reconnaît des structures glycanniques appartenant aux lipopolysaccharides bactériens. Les systèmes de reconnaissance de la lectine manifestant une bonne activité inhibitrice sont des glycopeptides du type complexe simultanément galactosylés et fucosylés, des oligosaccharides de lait humain ou des oligomannosides contenant 7,8 et 9 mannoses. L'inhibition se limite aux infections intestinales du cochon d'Inde, seul mammifère capable de libérer la lectine étudiée.

La composition selon l'invention est caractérisée par le fait qu'elle contient comme constituant actif un glycopeptide et/ou un oligosaccharide d'origine végétale ou un glycopeptide d'ovalbumine de formule:

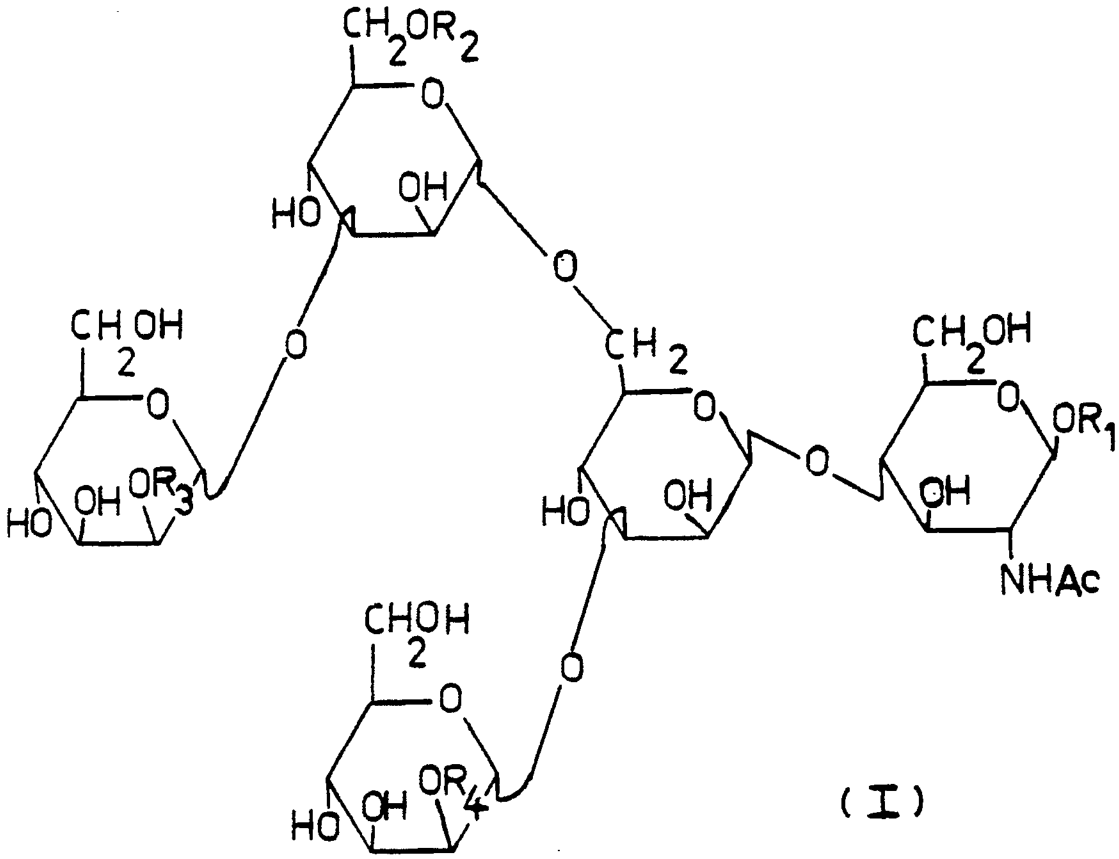

(I)

dans laquelle $R_1$ est un atome d'hydrogène , un résidu 4GlcNAc$\beta$1→ASN, ou un résidu

$$4GlcNAc\beta 1 \rightarrow ASN \genfrac{}{}{0pt}{}{R'}{R''} ,$$

dans lequel R' et R" sont les mêmes ou différents et représentent des résidus d'acides aminés ou des chaînes polypeptidiques; $R_2$, $R_3$ et $R_4$ sont les mêmes ou différents, et représentent des atomes d'hydrogène, des résidus de mannose ou des chaînes d'oligomannosides.

Comme bactéries pathogènes pourvues de fimbriae de type I on peut citer par exemple des souches pathogènes de Escherichia coli, Klebsiella pneumoniae, Salmonella typhimurium, Shigella flexneri, etc.

Dans la formule I) ci-dessus, 4GlcNAc$\beta$1→ASN signifie un cycle 2-désoxy-glucose relié à la glucosamine terminale en position 4, portant un groupe acétylamino en position 2 et relié en position 1 avec la configuration $\beta$ à l'asparagine, laquelle peut être substituée par des acides aminés ou des chaînes polypeptidiques.

Un groupe préféré, notamment pour son activité inhibitrice de l'adhérence des entérobactéries coliformes pathogènes, est constitué des glycopeptides de formule I ci-dessus dans laquelle $R_1$ est 4GlcNAc$\beta$1→ASN ou

$$4GlcNAc\beta 1 \rightarrow ASN \genfrac{}{}{0pt}{}{R'}{R''} ,$$

R' et R" ayant les significations ci-dessus, $R_3$ et $R_4$ sont des atomes d'hydrogène et $R_2$ est un atome d'hydrogène ou un résidu de mannose.

Un oligosaccharide préféré, notamment pour son activité inhibitrice de l'adhérence des entérobactéries coliformes pathogènes est celui de formule I dans laquelle $R_1$, $R_3$ et $R_4$ sont des atomes d'hydrogène et $R_2$ est un atome d'hydrogène ou un résidu de mannose.

Les composés de formule I peuvent être préparés en soumettant une glycoprotéine d'origine végétale à une digestion par un enzyme protéolytique, en transformant éventuellement les glycopeptides obtenus en

oligosaccharides par l'action d'une endo-β-N-acétylglucosaminidase H, puis en soumettant éventuellement les glycopeptides ou les oligosaccharides obtenus à une digestion contrôlée par une exo-α-mannosidase de manière à cliver préférentiellement les liaisons α1→2 entre deux résidus de mannose.

Toute farine de légumineuse connue pour contenir des glycoprotéines de réserve riches en oligomannosides peut servir de matériel de départ. On utilise avantageusement la farine dégraissée de soya ou de haricot.

De préférence, on utilise un isolat enrichi en glycoprotéine 7S de soya ou en glycoprotéine II de haricot, par extraction de la farine dégraissée à pH alcalin (8-9) puis précipitation sélective de la glycoprotéine à pH 4,5-5, par exemple de manière analogue à celle indiquée dans la littérature (réf. 2 et 4 ci-après).

Afin d'obtenir des glycopeptides, on soumet ensuite la fraction enrichie en glycoprotéine à une digestion par un enzyme protéolytique, par exemple de manière analogue à celle indiquée dans la littérature (réf. 3 ci-après).

La digestion enzymatique peut être effectuée à l'aide de n'importe quel enzyme protéolytique actif à pH acide, neutre ou alcalin. L'enzyme peut être d'origine fongique, microbienne (par exemple pronase, alcalase), végétale (par exemple bromeline, ficine, papaïne) ou animale (par exemple trypsine, pepsine, pancréatine).

Selon une première forme d'exécution du procédé destinée à préparer des oligosaccharides à partir des glycopeptides, on traite le digestat ci-dessus par une endo-β-N-acétylglucosaminidase H, destinée à cliver sélectivement la liaison β1→4 entre les deux résidus N-acetylglucosamine de la formule I, transformant ainsi $R_1$ en H.

Selon une variante préférée du procédé selon la première forme d'exécution, destinée à raccourcir les chaînes d'oligomannosides des oligosaccharides ci-dessus afin d'augmenter leur activité, on traite ces derniers de manière contrôlée par une exo-α-mannosidase, enzyme clivant préférentiellement les liaisons α1→2 entre deux résidus de mannose, par exemple de manière analogue à celle indiquée dans la littérature (réf. 5 ci-après).

Selon une seconde forme d'exécution du procédé' conduisant à des produits dont l'activité est plus grande que les précédents, on raccourcit les chaînes d'oligomannosides en traitant les glycopeptides précédents de manière controlée par une exo-α-mannosidase. On peut en variante préparer ensuite les oligosaccharides correspondants ci-dessus en traitant ces glycopeptides par une endo H. Les compositions selon l'invention peuvent être utilisées pour la prophylaxie, la thérapie ou le diagnostic de maladies infectieuses causées par les bactéries pourvues de fimbriae de type I, notamment les maladies gastro-intestinales causées par les entérobactéries coliformes comme par exemple Escherichia coli et se présenter sous une forme adaptée au mode d'administration.

Administré par exemple par voie orale ou entérale, le constituant actif peut se présenter sous forme de sirop, capsule, gélule, comprimé, dragée, de solution, suspension, émulsion ou de poudre reconstituable par addition d'un milieu aqueux, par exemple de préférence sous la forme d'un produit diététique, par exemple d'une poudre de lait.

Destiné à l'administration par voie parentérale, il peut se présenter sous forme d'une solution ou suspension stabilisée physiquement, stérile et apyrogène.

L'administration par voie topique, par exemple pour une application ophtalmologique peut être effectuée sous forme de solution, aérosol, pommade ou onguent.

Lorsque le constituant actif est destiné au diagnostic, à l'identification ou à l'isolement de bactéries pathogènes, il sera avantageusement couplé, de préférence par covalence à un support macromoléculaire.

Enfin les compositions selon l'invention peuvent être utilisées pour la désinfection des surfaces, par exemple sous forme de solution ou d'émulsion pour le traitement des verres de contact.

Dans ces compositions le constituant actif peut représenter 0,1 à 90 % en poids.

Les exemples suivants illustrent l'invention. Dans ceux-ci, les parties et pourcentages sont pondéraux sauf indication contraire.

Dans les exemples 1 à 3 ci-après, la preuve de la structure des constituants actifs de formule I découle des caractéristiques suivantes :

a) l'analyse de la composition glucidique des glycoprotéines de départ ne révèle la présence que de deux constituants monosaccharidiques : le mannose et la glucosamine. Les oligosaccharides formés de ces deux constituants sont liés à la chaîne polypeptidique par l'azote d'une asparagine (liaison N-glycosidique), la partie "endo" de ces oligosaccharides correspondant alors à la structure :

Manα1 → 6
Manβ1 → 4GlcNAcβ1 → 4GlcNAcβ1 → ASN
Manα1 → 3

b) Le fait que tous les substrats glycopeptidiques soient entièrement hydrolysés par l'Endo-β-N-acétylglucosaminidase H (Endo H) prouve qu'ils correspondent tous à la structure minimum requise pour une telle digestion enzymatique (réf. 6 ci-après)

Manα1 → 3Manα1 → 6Manβ1 → 4GlcNAcβ1 → 4GlcNAc

La superposition des deux structures ci-dessus conduit à l'élaboration de la structure générale proposée.

Exemple 1

a) Glycoprotéine : On prépare une fraction enrichie en glycoprotéine 7S à partir de farine de soya dégraissée par extraction à pH 8,0 avec une solution 0,5 mMolaire de $Na_2SO_3$, précipitation à pH 5,7 permettant d'écarter une première fraction (riche en 11S) et une seconde précipitation à pH 4,5, suivie de deux lavages au même pH qui donne accès à la fraction enrichie en 7S.

b) Glycopeptides : On conduit la digestion enzymatique de la partie protéinique à partir de 30 g de protéine, dans deux litres de solution tampon Tris-HCl (0,05 Molaire, pH 8,0), en présence de toluène, pendant 24 heures à 40° C à l'aide de 600 mg de pronase E (Merck AG), puis de 300 mg additionnels d'enzyme pendant 48 heures. On isole les glycopeptides après filtration de cette solution, passage de l'éluat sur résine Dowex 50W-X8® (forme $H^+$), lavage de la résine à l'eau distillée jusqu'à disparition des glucides dans les eaux de lavage, et neutralisation des fractions combinées avec une résine Amberlite IRA 400®

(forme $CO_3^{--}$).

On concentre et on lyophilise la solution finale et le produit peut être finalement purifié par fractionnement sur une colonne de Sephadex G-25®. Le rendement global du procédé est de 88% (basé sur la quantité de mannose finalement présent dans le mélange glycopeptidique). L'analyse de la composition glucidique du mélange obtenu ci-dessus (par la méthode décrite dans la réf. 7 ci-après) a révélé la présence d'une moyenne de 7,6 unités de mannose pour deux unités de N-acétyl-glucosamine.

c) Oligosaccharides : Les glycopeptides isolés ci-dessus se sont avérés complètement digestibles par l'endo-β-N-acétylglucosaminidase H (Endo H, Seikagaku Kogyo Co. Ltd.), cette dernière propriété fournissant la base du procédé utilisé pour l'obtention des oligosaccharides correspondants : on dissout un échantillon glycopeptidique contenant 7,5 mg d'oligomannoside dans 10 ml de solution tampon citratephosphate (pH 6,0, 10 mMolaire) et on ajoute à cette solution 100 milliunités d'Endo H (unité définie par le fabricant) ainsi que 0,5 ml de toluène. Après une incubation de 24 heures à 37° C, on dénature l'enzyme par chauffage. L'hydrolysat ainsi obtenu peut être utilisé sous cette forme (brute) pour des tests d'hémagglutination (voir exemple 4 ci-après). Le même hydrolysat peut également être traité par une résine Amberlite MB 3® (forme $H^+$ et $OH^-$), dans le but d'isoler les oligosaccharides ainsi libérés : après addition, agitation et décantation de la résine, on lave cette dernière à l'eau distillée jusqu'à disparition des glucides dans les eaux de lavage et on concentre les fractions combinées. L'analyse des oligosaccharides ainsi purifiés a révélé la présence de 7 à 8 unités de mannose par unité de N-acétylglucosamine, pour un produit contenant 85% de mannose, le rendement glogal de l'hydrolyse enzymatique et de l' isolement des oligosaccharides étant pratiquement quantitatif. L'analyse par chromatographie sur couche mince à haute performance (HPTLC) du produit brut de la digestion à l'Endo H et du mélange des oligosaccharides purifiés conduit à des résultats similaires : l'enzyme libère trois produits différents correspondant aux structures GlcNAc-$(Man)_6$ (18%), GlcNAc-$(Man)_7$ (26%) et GlcNAc- $(Man)_8$ (56%) (Man signifiant un résidu de mannose), ces hydrates de carbone étant totalement

libérés depuis les glycopeptides de départ.

Exemple 2

a) Glycoprotéine : On prépare une fraction enrichie en glycoprotéine II à partir de farine de haricot blanc (Phaseolus vulgaris) moulu dégraissée par extraction à pH 9,0 et dialyse contre l'eau acidifiée à pH 5,0 résultant en une précipitation de la fraction glycoprotéinique qui est séparée et à nouveau mise en solution à pH 8,0. Après deux centrifugations, et lyophylisation des surnageants combinés, on obtient la fraction riche en glycoprotéine II.

b) Glycopeptides : On conduit la digestion enzymatique de la partie protéinique exactement comme depuis la glycoprotéine 7S du soya (exemple 1b ci-dessus). L'analyse de la composition glucidique du mélange obtenu (réf. 7 ci-après) a révélé la présence d'une moyenne de 7,8 unités de mannose pour deux unités de N-acétyl-glucosamine.

c) Oligosaccharides : Les glycopeptides isolés ci-dessus se sont avérés complètement digestibles par l'Endo-H. On conduit l'hydrolyse enzymatique et l'isolation des oligosaccharides exactement comme depuis les glycopeptides du soya (voir exemple 1c ci-dessus). L'analyse par HPTLC a révélé dans ce cas la libération de 5 produits différents, correspondant aux structures GlcNAc-$(Man)_5$ (5%), GlcNAc-$(Man)_6$ (10%), GlcNAc-$(Man)_7$ (26%), GlcNAc-$(Man)_8$ (15%) et GlcNAc-$(Man)_9$ (44%).

Exemple 3

Dans le but de générer des glycopeptides et des oligosaccharides à structure réduite à partir des produits des exemples 1 et 2, on tire profit de la propriété de l'α-mannosidase (de canavalie, Canavalia, Sigma Chemical Company), de cliver les liaisons Manα1→2 Man préferentiellement aux liaisons Manα1→3Man.

On dissout un échantillon de glycopeptides contenant 350 mg d'oligomannoside provenant de la glycoprotéine 7S du soya (exemple 1b ci-dessus) dans 75 ml de solution tampon citrate (0,01 Molaire, pH 4,5) et on ajoute à cette solution 190 unités d'α-mannosidase (de canavalie, unité défine par le fabricant). Après une incubation d'1 h. à 25° C, on ébouillante le mélange, on le refroidit et on le filtre. On lyophilise ensuite le filtrat. Le lyophilisat peut être utilisé comme tel (brut) pour des tests d'hémagglutination (voir exemple 4 ci-après). Il peut également être purifié sur colonne de Sephadex G-25®, purification permettant la séparation effective du mélange glycopeptidique et du mannose libéré par l'enzyme.

L'analyse de la composition glucidique du produit (réf. 7 ci-après) a révélé la présence de 4,8 unités de mannose pour deux de N-acétyl-glucosamine.

L'analyse par HPTLC des produits obtenus comme ci-dessus, mais depuis les oligosaccharides d'origine végétale correspondants (exemples 1c et 2c ci-dessus) confirme qu'un composé correspondant à la formule GlcNAc-$(Man)_5$ est bien le constituant principal des mélanges obtenus.

Exemple 4

Inhibition d'hémagglutination des érythrocytes de cobaye par des souches adhérentes de E. Coli, en présence de glycopeptides et d'oligosaccharides.

Deux souches de bactéries adhérentes E. Coli ont été utilisées systématiquement dans les tests d'hémagglutination et d'inhibition d'hémagglutination : un isolat clinique d'E. Coli 16375 (Univ.-Klinik für Kinderheilkunde, Innsbruck) et la souche d'E. Coli 0119.K69,L74-30 (K69). Ces bactéries ont été lavées avec de l'eau saline (0,9% NaCl) et les suspensions ont été ajustées à une concentration de $10^9$ bactéries/ml (par mesure de densité optique).

Les érythrocytes de cobaye ont été suspendus à une concentration de 1% dans de l'eau saline.

Les tests d'hémagglutination et d'inhibition d'hémagglutination ont été effectués en mélangeant 25μl (microlitre) de la suspension bactérienne, 50μl de la suspension d'érythrocytes et 25μl d'une solution saline (respectivement ne contenant pas ou contenant un inhibiteur, dans ce dernier cas plusieurs concentrations différentes étant testées en série). Les lectures ont été effectuées après deux heures d'immobilisation à 4° C.

Les résultats sont présentés dans le tableau I ci-après :

## TABLEAU I

| Inhibiteur | Inhibition d'hemagglutination d'erythrocytes de cobaye: Concentration[a)] (ppm d'oligomannoside) E.Coli 16375 | E.Coli K 69 | Concentration[b)] (μ-Molarité) E.Coli 16375 | E.Coli K 69 | Activité relative à l'α-MM |
|---|---|---|---|---|---|
| Méthyl-α-D-mannoside (α-MM) | 125 | 60 | 650 | 325 | 1 |
| Glycopeptides / Endo-H digestat (brut) / Oligosaccharides purifiés — De la glycoprotéine 7S des graines de soya | 130/150 | 65/75 | 100 | 50 | 6,5 |
| Glycopeptides / Endo-H digestat (brut) / Oligosaccharides purifiés — De la glycoprotéine II des graines de haricot | 125 | 60 | 90 | 45 | 7 |
| Mélange des glycopeptides de l'ovalbumine c) | 40/60 | 20/30 | 70 | 35 | 10 |
| GP IV | 95 | n.t. | 90 | n.t. | 7,5 |
| GP V | 10/15 | 5 | 12 | 6 | 60 |
| Oligosaccharides (de la 7S du soya) digérés (1h.) à l'α-mannosidase | 10/25 | 10/15 | 18 | 12 | 30 |
| Oligosaccharides (de la glycopr. II du haricot) digérés (1h.) à l'α-mannosidase | 10/25 | 10 | 18 | 12 | 30 |
| Manα1→3Manβ1→4GlcNAc (d'origine synthéthique)[d)] | 7,5 | 5,5 | 20 | 15 | 25 |
| Glycopeptides ( de la 7S du soya) digérés (1h) à l'α-mannosidase | 12/18 | 6/9 | 15 | 7,5 | 40 |
| Glycopeptides (de la glycopr. II du haricot) digérés (1h.) à l'α-mannosidase | 12/18 | 6/9 | 15 | 7,5 | 40 |

Tableau I, légende :

a) Concentration minimum d'inhibiteur dans le mélange final, résultant en une inhibition complète de l'hémagglutination.

b) Expression calculée de la concentration de l'inhibiteur, depuis la formule (moyenne dans le cas de mélanges) déduite de l'analyse de la composition glucidique du produit.

c) Les produits nommés GP IV et V correspondent aux hydrates de carbone liés à une asparagine, obtenus, séparés et nommés conformément à la littérature (réf. 5 ci-après). Le mélange des glycopeptides de l'ovalbumine correspond à celui décrit (réf. 5 ci-après).

d) Produit synthétique aimablement offert par le Prof. Dr. Hans Paulsen (Institut für Organische Chemie und Biochemie der Universität, Hamburg).

n.t. signifie non testé.

D'autre expériences ont montré que les glycopeptides végétaux inhibent de manière comparable l'hémagglutination des érythrocytes de cobaye provoquée par les souches entéropathogènes E.Coli 086.K61,B74-10 et 0111.K58,B75-44.

Exemple 5

Adhérence et inhibition d'adhérence d'E. Coli entéropathogènes 16375 sur cellules buccales humaines.

Des cellules ont été recueillies par frottis de la bouche d'un membre du personnel de laboratoire, lavées quatre fois avec une solution d'eau physiologique tamponée au phosphate (NaCl 0,15 Molaire, phosphate 0,01 Molaire, (PBS)), et finalement diluées jusqu'à une concentration de $10^6$ cellules/ml.

Les bactéries entéropathogènes E. Coli de l'isolat clinique 16375 ont été lavées deux fois au PBS et diluées jusqu'à une concentration de $2.10^9$ bactéries/ml.

Les incubations ont été effectuées en mélangeant 500µl de la suspension cellulaire, 250µl de la suspension bactérienne et 250µl de PBS (pour la mesure de l'adhérence) ou d'inhibiteur (une série de concentrations différentes étant testées pour la mesure de l'inhibition). Les mélanges ont été effectués en rotation lente pendant 30 minutes à température ambiante. Quatre lavages au PBS (5 ml) ont alors précédé les collectes de frottis et les colorations au Gram. Le nombre de bactéries adhérées par cellule a été compté sous microscope optique, 50 cellules par test étant analysées. Les résultats sont regroupés dans le Tableau II ci-après :

TABLEAU II

| Inhibiteur | Concentration (ppm équivalent mannoside) | % d'inhibition d'adhérence |
|---|---|---|
| Méthyl-α-D-mannoside : | 100 | 70 |
| | 50 | 45 |
| | 10 | 35 |
| Glycopeptides de la glycoprotéine 7S du soya (ex. 1b). | 100 | 65 |
| | 50 | 30 |
| | 10 | 25 |
| Glycopeptides de la 7S du soya, digérés (1h) à l'α-mannosidase (ex. 3). | 100 | 90 |
| | 25 | 77 |
| | 5 | 21 |

Exemple 6

Immobilisation de glycopeptides sur support solide

Les glycopeptides produits par les digestions à la pronase (exemples 1b et 2b) peuvent être fixés sur gel de Sepharose, suivant la méthode ci-après : 2 g de Sepharose 6MB® activité au CNBr (Pharmacia Fine Chemicals) ont été lavés avec une solution d'HCl (1 mMolaire, 400 ml) et filtrés. Parallèlement, 63 mg (poids sec, contenant 25 mg d'oligomannoside) de glycopeptides provenant de la glycoprotéine II du haricot (exemple 2b) ont été dissouts dans une solution tampon $NaHCO_3$ (0,1 Molaire, pH 8,3) contenant du NaCl (0,5 Molaire). Le mélange de la solution glycopeptidique et du gel en suspension a été effectué en rotation lente pendant deux heures à température ambiante. Des lavages successifs au tampon $NaHCO_3$/NaCl, à une solution d'éthanolamine (deux heures à température ambiante), à nouveau au tampon $NaHCO_3$/NaCl, avec une solution tampon acétate (0,1 Molaire, pH 4,0) contenant du NaCl (0,5 Molaire) et finalement à nouveau au tampon $NaHCO_3$/NaCl, donnent accès au gel couplé aux glycopeptides. Le rendement de la réaction a été de 48% de fixation (basé sur le dosage du mannose).

Exemple 7

Test de diagnostic pour l'identification de bactéries munies d'accepteurs spécifiques pour les structures des constituants actifs.

a) Des bactéries sont mélangées avec des érythrocytes de cobaye pour un test d'hémagglutination, conformément, à l'exemple 4. Parallèlement, on effectue un mélange similaire avec l'addition d'une solution d'un des produits biologiquement actifs (en concentration suffisante pour provoquer une inhibition complète, conformément au tableau I). Une lecture confirmant l'hémagglutination d'érythrocytes par la suspension bactérienne, ainsi que son inhibition par l'addition de l'un des constituants actifs fournira la preuve que les bactéries possèdent des accepteurs spécifiques pour la structure de ce constituant.

b) Alternativement, un mélange d'une suspension bactérienne et du gel de Sepharose couplé aux glycopeptides (exemple 6) peut être effectué sur une plaque de microscope. Après 15 minutes

d'incubation, l'analyse par microscopie optique révèle que si les bactéries possèdent les accepteurs spécifiques, elles couvrent les particules du gel, tandis que dans le cas contraire, les mêmes particules ne fixent pas de bactéries.

Exemple 8

Isolement de bactéries munies d'accepteurs spécifiques pour les structures des constituants actifs.

Le gel de Sepharose couplé aux glycopeptides obtenu selon l'exemple 6 est chargé sur une colonne. Un mélange de bactéries est passé à travers cette colonne, les bactéries possèdant des accepteurs spécifiques pour les glycopeptides couplés au gel étant retenues, tandis que les autres bactéries sont éluées directement. Après rinçage, un tampon contenant l'un des constituants actifs est utilisé pour éluer les bactéries munies des accepteurs spécifiques sous une forme pure.

REFERENCES

1. M. Firon, I. Ofek and N. Sharon, Carbohydr. Res. 120 (1983), 235-249.
2. M. Shemer, H.L. Creinin, R.E. McDonald and W.E. Irwin, Cereal Chem. 55 (1978), 383-391.
3. F. Yamauchi, M. Kawase, M. Kanbe and K. Shibazaki, Agr. Biol. Chem. 39 (1975), 873-878.
4. A. Pusztai and W.B. Watt, Biochem. Biophys. Acta 207 (1979), 413-431.
5. T. Tai, K. Yamashita, M. Ogata-Arakawa, N. Koide, T. Muramatsu, S. Iwashita, Y. Inoue and A. Kobota, J. Biol. Chem. 250 (1975), 8569-8575.
6. A. Tarentino, R.B. Trimble and F. Maley, Methods in Enzymology (V. Ginsburg, ed., Academic Press, New York), 50 (1978) 574-580.
7. J.R. Neeser and T.F. Schweizer, Anal. Biochem. (1984).

**Revendications**

**1.** Composition anti-bactérienne relative à des bactéries pathogènes pourvues de fimbriae de type I, caractérisée par le fait qu'elle contient comme constituant actif un glycopeptide et/ou un oligosaccharide d'origine végétale ou un glycopeptide d'ovalbumin de formule :

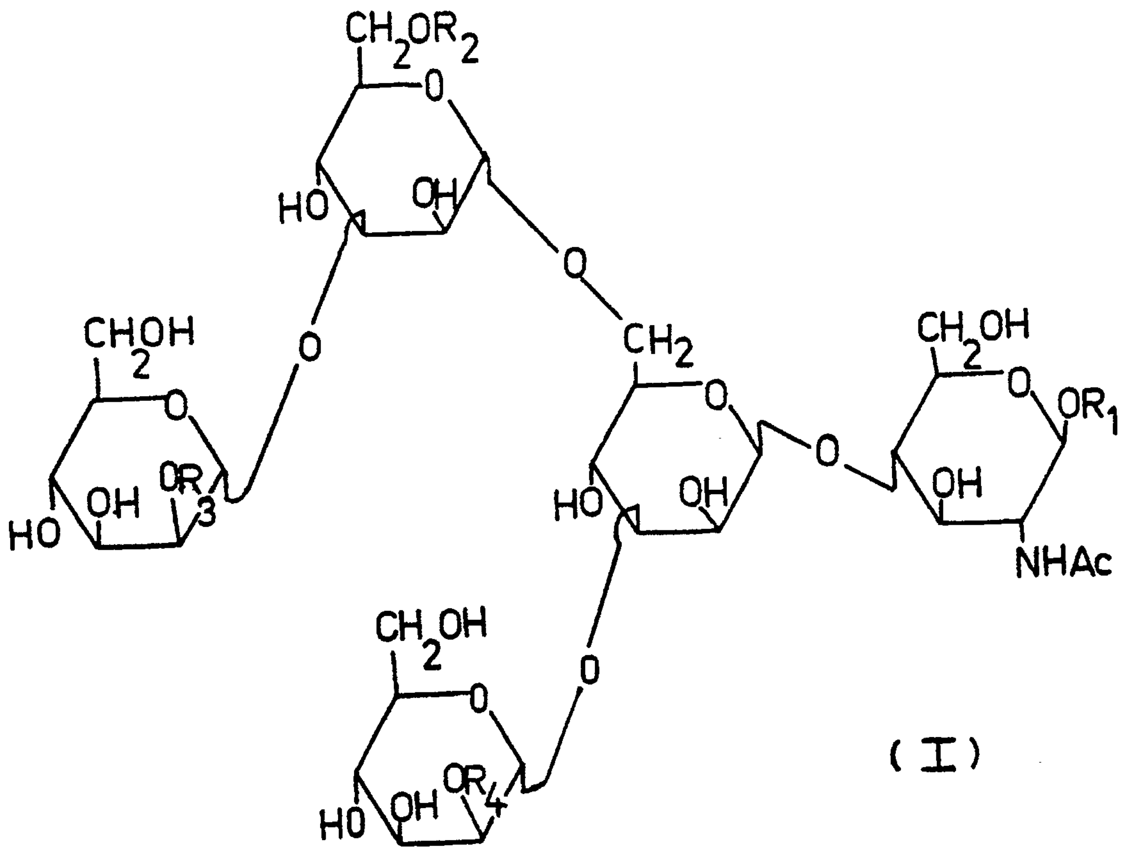

(I)

dans laquelle $R_1$ est un atome d'hydrogène, un résidu 4GlcNAc$\beta$1→ASN, ou un résidu

$$4GlcNAc\beta 1 \rightarrow ASN <^{R'}_{R''} ,$$

dans lequel R' et R" sont les mêmes ou différents et représentent des résidus d'acides aminés ou des chaînes polypeptidiques; $R_2$, $R_3$ et $R_4$ sont les mêmes ou différents, et représentent des atomes d'hydrogène, des résidus de mannose ou des chaînes d'oligomannosides.

**2.** Composition selon la revendication 1, caractérisée par le fait qu'elle contient comme constituant actif un composé de formule I, dans laquelle $R_1$ est un résidu 4GlcNAc$\beta$1→ASN ou un résidu

$$4GlcNAc\beta 1 \rightarrow ASN <^{R'}_{R''} ,$$

dans lequel R' et R" sont semblables ou différents et représentent des résidus d'acides aminés ou des chaînes polypeptidiques, $R_3$ et $R_4$ représentent des atomes d'hydrogène et $R_2$ est un atome d'hydrogène ou un résidu de mannose.

**3.** Composition selon la revendication 1, caractérisée par le fait qu'elle contient comme constituant actif le composé de formule I, dans laquelle $R_1$, $R_3$ et $R_4$ sont des atomes d'hydrogène et $R_2$ est un atome d'hydrogène ou un résidu de mannose.

**4.** Composition selon la revendication 1, caractérisée par le fait qu'elle contient un glycopeptide de formule I dans laquelle $R_1$ représente un résidu 4GlcNAc$\beta$1→ASN ou un résidu

$$4GlcNAc\beta1 \rightarrow ASN <^{R'}_{R''} ,$$

dans lesquels R', R" et $R_1$, $R_2$, $R_3$ et $R_4$ ont les significations indiquées dans la revendication 1, couplé de préférence par covalence à un support macromoléculaire.

5. Composition selon l'une des revendications 1 à 3, sous la forme d'un produit diététique, de préférence sous la forme d'une poudre de lait.

6. Procédé de préparation d'une composition selon la revendication 4, caractérisé par le fait qu'on fait réagir un support macromoléculaire activé avec une solution de glycopeptide.

7. Utilisation d'une composition selon l'une des revendications 1 à 4 pour l'identification et/ou l'isolement de bactéries spécifiques pourvues de fimbriae de type I.

8. Utilisation d'une composition selon l'une des revendications 1 à 3 pour la désinfection des surfaces.

**Claims**

1. An antibacterial composition for pathogenic bacteria provided with type I fimbriae, characterized in that it contains as active constituent a glycopeptide and/or an oligosaccharide of vegetable origin or a glycopeptide of ovalbumin corresponding to the following formula:

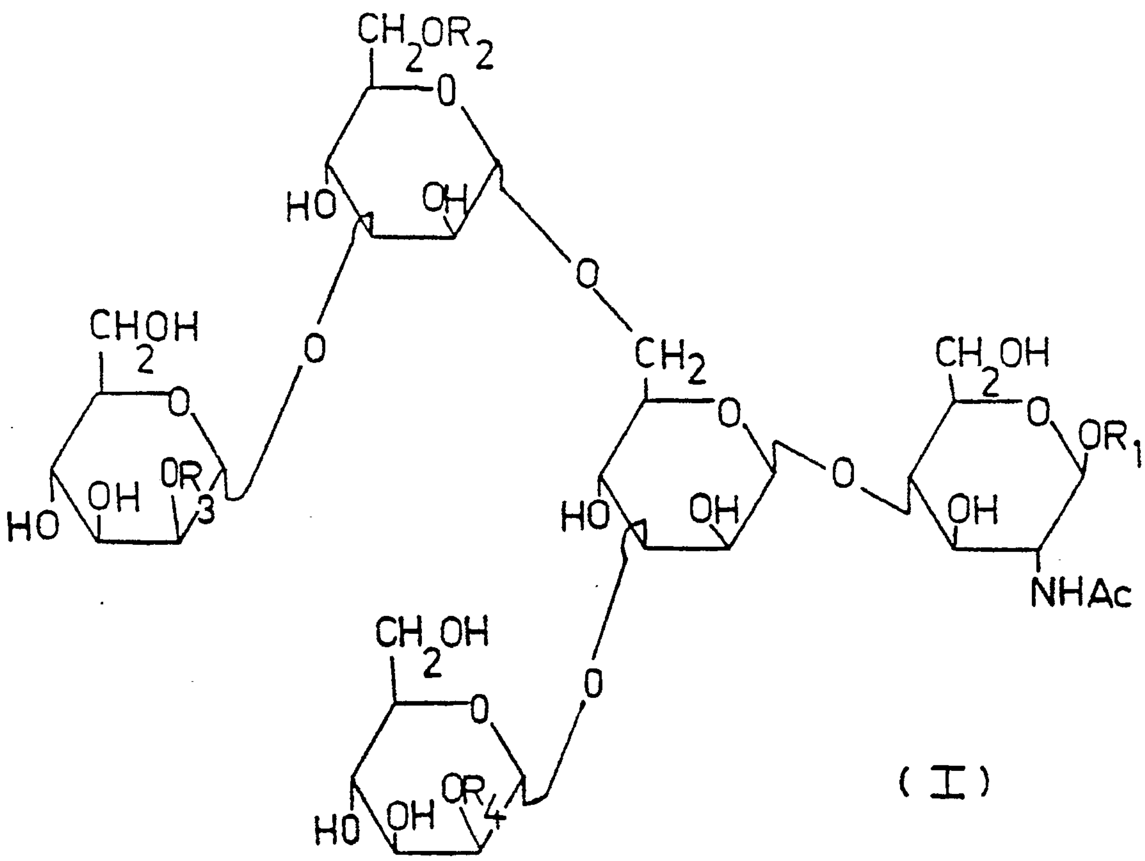

(I)

in which $R_1$ is a hydrogen atom, a residue 4GlcNAcβ1→ASN or a residue

$$4GLcNAc\beta1 \rightarrow ASN <^{R'}_{R''} ,$$

where R' and R" are the same or different and represent aminoacid residues or polypeptide chains; $R_2$, $R_3$ and $R_4$ are the same or different and represent hydrogen atoms, mannose residues or oligomannoside chains.

2. A composition as claimed in Claim 1, characterized in that it contains as active constituent a compound of formula I in which $R_1$ is a residue 4GlcNAc$\beta$1→ASN or a residue

$$4GlcNAc\beta 1 \rightarrow ASN \begin{matrix} \diagup R' \\ \diagdown R'' \end{matrix} ,$$

where R' and R" are similar or different and represent aminoacid residues or polypeptide chains; $R_3$ and $R_4$ represent hydrogen atoms and $R_2$ is a hydrogen atom or a mannose residue.

3. A composition as claimed in Claim 1, characterized in that it contains as active constituent the compound of formula I in which $R_1$, $R_3$ and $R_4$ are hydrogen atoms and $R_2$ is a hydrogen atom or a mannose residue.

4. A composition as claimed in Claim 1, characterized in that it contains a glycopeptide of formula I in which $R_1$ is a residue 4GlcNAc$\beta$1→ASN or a residue

$$4GlcNAc\beta 1 \rightarrow ASN \begin{matrix} \diagup R' \\ \diagdown R'' \end{matrix} ,$$

where R', R" and $R_1$, $R_2$, $R_3$ and $R_4$ have the meanings defined in Claim 1, coupled preferably by covalent bonding to a macromolecular support.

5. A composition as claimed in any of Claims 1 to 3 in the form of a dietetic product, preferably in the form of a milk powder.

6. A process for preparing the composition claimed in Claim 4, characterized in that an activated macromolecular support is reacted with a glycopeptide solution.

7. The use of the composition claimed in any of Claims 1 to 4 for identifying and/or isolating specific bacteria provided with type I fimbriae.

8. The use of the composition claimed in any of Claims 1 to 3 for disinfecting surfaces.

## Patentansprüche

1. Gegenüber pathogenen, mit Fimbrien vom Typ I versehenen Bakterien antibakterielle Zusammensetzung, dadurch gekennzeichnet, daß die als wirksamen Bestandteil ein Glycopeptid und/oder ein Oligosaccharid pflanzlicher Herkunft oder ein Eialbumin-Glycopeptid der Formel:

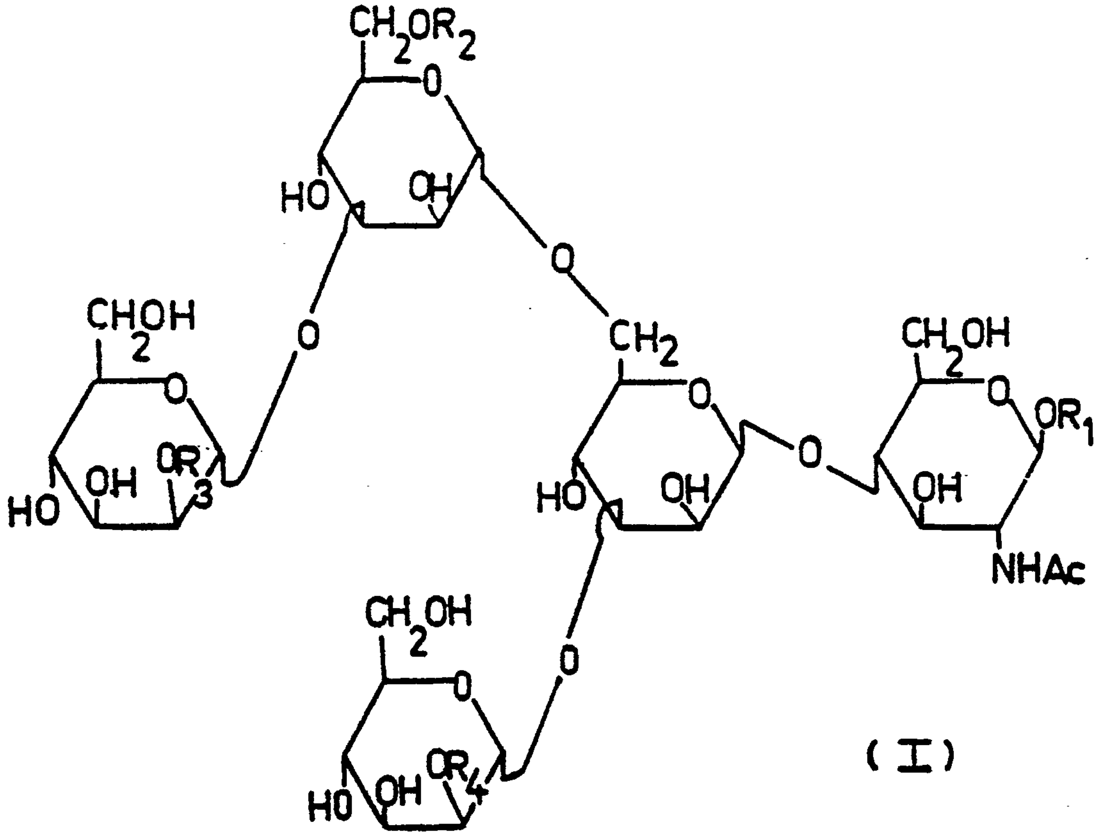

(I)

enthält, worin $R_1$ ein Wasserstoffatom, einen 4GLcNAc$\beta$1→ASN-Rest oder einen

$$4GlcNAc\beta 1 \rightarrow ASN{<}^{R'}_{R''}\text{-Rest}$$

bedeutet, worin R' und R" gleich oder verschieden sind und Aminoreste oder Polypeptidketten-Reste darstellen; $R_2$, $R_3$ und $R_4$ gleich oder verschieden sind und Wasserstoffatome, Mannosereste oder Oligomannosidketten-Reste bedeuten.

2. Zusammensetzung nach Anspruch 1, dadurch gekennzeichnet, daß sie als wirksamen Bestandteil eine Verbindung der Formel I enthält. worin $R_1$ einen 4GlcNAc$\beta$1→ASN-Rest oder einen

$$4GlcNAc\beta 1 \rightarrow ASN{<}^{R'}_{R''}\text{-}$$

Rest darstellt, worin R' und R" gleich oder verschieden sind und Aminosäurereste oder Polypeptidketten-Reste bedeuten, $R_3$ und $R_4$ Wasserstoffatome darstellen und $R_2$ ein Wasserstoffatom oder einen Mannoserest bedeutet.

3. Zusammensetzung nach Anspruch 1, dadurch gekennzeichnet, daß sie als wirksamen Bestandteil die Verbindung der Formel I enthält, worin $R_1$, $R_3$ und $R_4$ Wasserstoffatome bedeuten und $R_2$ ein Wasserstoffatom oder einen Mannoserest darstellt.

4. Zusammensetzung nach Anspruch 1, dadurch gekennzeichnet, daß sie ein Glycopeptid der Formel I enthält, worin $R_1$ einen 4GlcNAc$\beta$1→ASN Rest oder einen

$$4GlcNAc\beta 1 \rightarrow ASN{<}^{R'}_{R''}\text{-Rest}$$

bedeutet, worin R', R" und $R_1$, $R_2$, $R_3$ und $R_4$ die in Anspruch 1 angegebenen Bedeutungen aufweisen, gekuppelt, vorzugsweise durch kovalente Bindung, an einen makromolekularen Träger.

**5.** Zusammensetzung nach einem der Ansprüche 1 bis 3 in Form eines diätetischen Produktes, vorzugsweise in Form eines Milchpulvers.

**6.** Verfahren zur Herstellung einer Zusammensetzung nach Anspruch 4, dadurch gekennzeichnet, daß man einen aktivierten makromolekularen Träger mit einer Glycopeptidlösung umsetzt.

**7.** Verwendung einer Zusammensetzung nach einem der Ansprüche 1 bis 4 zur Identifizierung und/oder Isolierung von spezifischen, mit Fimbrien vom Typ I versehenen Bakterien.

**8.** Verwendung einer Zusammensetzung nach einem der Ansprüche 1 bis 3 zur Desinfektion von Oberflächen.